# FASCICULE DE BREVET EUROPEEN

(11) **EP 4 085 246 B1**
(45) Date de publication et mention de la délivrance du brevet: **08.05.2024**
(21) Numéro de dépôt: 20845780.4
(22) Date de dépôt: 21.12.2020
(51) Int. Cl.: G01N 21/85, G01N 21/47, G01N 21/53, G01N 15/02, G01N 15/06

(54) **DISPOSITIF DE DETECTION DE PRESENCE DE POLLENS DANS L'AIR, ET PROCEDE DE DETECTION CORRESPONDANT**
VORRICHTUNG ZUR DETEKTION DER ANWESENHEIT VON POLLEN IN DER LUFT UND ENTSPRECHENDES NACHWEISVERFAHREN
DEVICE FOR DETECTING THE PRESENCE OF POLLEN IN THE AIR, AND CORRESPONDING DETECTION METHOD

(30) Priorité: 30.12.2019 FR 1915708
(43) Date de publication de la demande: 09.11.2022
(73) Titulaire: Lify Air, 45100 Orléans (FR); CENTRE NATIONAL DE LA RECHERCHE SCIENTIFIQUE, 75794 Paris Cedex 16 (FR); Université d'Orléans, 45100 Orléans (FR)
(72) Inventeur: RICHARD, Jérôme, 45560 Saint Denis en Val (FR); LAUTHIER, Johann, 45800 Saint Jean de Braye (FR); RENARD, Jean-Baptiste, 45000 Orléans (FR)
(74) Mandataire: Boüan du Chef du Bos, Louis-Paterne
(86) Numéro de dépôt international: PCT/FR2020/000283
(87) Numéro de publication internationale: WO 2021/136889

(56) Documents cités:
- EP-A1- 1 281 951
- EP-A1- 3 392 644
- EP-A1- 3 413 034
- CN-A- 106 018 193
- US-A1- 2004 066 513
- CROUZY BENOÎT ET AL: "All-optical automatic pollen identification: Towards an operational system", ATMOSPHERIC ENVIRONMENT, PERGAMON, GB, vol. 140, 2 juin 2016 (2016-06-02), pages 202-212, XP029631512, ISSN: 1352-2310, DOI: 10.1016/J.ATMOSENV.2016.05.062

## Description

### Domaine de l'invention

La présente invention concerne un dispositif de détection de présence de pollens dans l'air.

L'invention concerne également un procédé de détection de présence de pollens dans l'air.

### Art antérieur

Les particules, ou grains de pollens, quand ils sont disséminés dans l'air, constituent une cause majeure de réaction allergique. Ils peuvent ainsi causer, pour les personnes sensibles, des symptômes se présentant généralement sous forme de rhinite allergique, souvent accompagnée d'une conjonctivite. De telles réactions allergiques peuvent être extrêmement préjudiciables aux personnes qui les subissent. Par ailleurs, elles peuvent entraîner l'apparition d'un asthme chez ces personnes.

Les personnes allergiques ne sont pas sensibles à tous les types de pollens. Généralement, une personne peut être sensible à un type de pollen, ou à plusieurs types. En France, les pollens de certains arbres, comme le bouleau, le cyprès, le frêne ou le noisetier, de certaines graminées comme les céréales ou les dactyles, ou encore de certains herbacés comme l'oseille ou l'ambroisie, sont particulièrement connus pour déclencher des réactions allergiques chez des personnes sensibles.

Pour les personnes sensibles, il est particulièrement utile de connaître les lieux et les périodes où apparaissent les pollens qui déclenchent chez eux des réactions allergiques. Cette connaissance leur permet de prendre des mesures pour réduire le risque de réaction allergique, comme par exemple des mesures d'évitement des zones concernées, de confinement à l'intérieur de bâtiments quand l'air comprend une concentration trop importante du pollen incriminé, de port d'un masque respiratoire, de prise préventive d'un médicament tel qu'un antihistaminique, etc.

Pour leur permettre d'obtenir ces informations, il existe des calendriers permettant de connaître, de façon approximative, les périodes de dissémination de chaque pollen, et des cartographies permettant de connaître les zones dans lesquelles apparaissent ces pollens. Ces données sont cependant très imprécises. Il existe aussi des capteurs destinés à connaître la concentration de particules de pollens dans l'air, au cours d'une période déterminée. Les capteurs utilisés le plus couramment sont soit de type gravimétrique, soit de type volumétrique.

On connaît ainsi des appareils dits gravimétriques, qui recueillent les grains de pollen qui se déposent par gravité sur un support horizontal. Ces appareils, par exemple du type connu sous le nom de « Durham », présentent un rendement de récupération relativement faible. En conséquence, leurs mesures sont réputées être peu précises, surtout quand elles portent sur des périodes de mesure relativement courtes.

On connaît également des appareils dits volumétriques, par exemple du type connu sous le nom de « Hirst », dans lesquels un débit d'air est projeté sur un support adhésif, les particules contenues dans l'air se collant sur ce support adhésif. Il est ensuite possible d'observer, en microscopie optique, les particules de pollens ainsi piégées.

Dans d'autres appareils, connus sous le nom de « Cour », un flux d'air passe à travers un filtre qui récupère les particules de pollens. Le filtre est ensuite dissous en laboratoire pour l'observation du pollen en microscopie optique.

De tels appareils, qui constituent les équipements de mesure de référence à ce jour, nécessitent de nombreuses manipulations. Ainsi, le support adhésif ou le filtre ayant recueilli les particules de pollens doit être relevé régulièrement puis être analysé en laboratoire. La précision des mesures dépend de la fréquence des relevés, et le résultat pour une période donnée ne peut être obtenu que plusieurs heures après la fin de cette période.

Les mesures de pollens obtenues par de telles méthodes ne permettent donc pas de fournir aux personnes sensibles des informations réellement pertinentes pour leur permettre d'éviter une crise allergique. En effet, ces personnes ne peuvent obtenir les informations concernant l'apparition de pollens dans l'air que plusieurs heures après cette apparition, et donc, le plus souvent, après le déclenchement de leur réaction allergique. Le manque d'informations en temps réel sur la présence de pollens dans l'air est donc préjudiciable pour ces personnes sensibles.

De nombreuses autres méthodes de mesure de la concentration de pollens dans l'air ont été proposées. Elles présentent cependant des inconvénients, tels qu'un coût ou un encombrement importants des appareils nécessaires, une difficulté à discriminer les types de pollens observés, ou encore un délai important entre la période d'observation et l'obtention des résultats. Ces méthodes alternatives sont donc peu utilisées. Il est également connu de CN106018193 A de déterminer la présence et la concentration de particules polluantes dans l'air en utilisant des mesures de diffusion lumineuse dans au moins quatre directions différentes. En outre, selon EP3413034 A1, des mesures de fluorescence et de diffusion optique combinées avec des données de location géographique des mesures permettent d'identifier et compter les différents pollens présents dans un endroit donné.

Du fait de ces difficultés à détecter et mesurer les particules de pollens présentes dans l'air, la densité géographique des points de mesure est très faible, et les résultats ne sont connus que longtemps après la période de mesure. Les informations obtenues sont donc peu utilisables par les personnes sensibles.

### Objectifs de l'invention

La présente invention a pour objectif de pallier ces inconvénients de l'art antérieur.

En particulier, l'invention a pour objectif d'offrir un dispositif de détection de pollens permettant une détection et une mesure fiables, efficaces et rapides des particules de pollens présentes dans l'air.

Selon au moins certains de ses modes de réalisation, l'invention a également pour objectif d'offrir un tel dispositif qui permette d'effectuer une mesure précise de la quantité de chaque type de pollen présent dans l'air.

L'invention a également pour objectif de fournir un tel dispositif qui permette d'identifier, avec une fiabilité accrue, les types de pollens présents dans l'air.

Dans au moins certains des modes de réalisation, l'invention a également pour objectif de permettre la communication à des utilisateurs de la quantité de pollens présents dans l'air en un point donné, dans un délai très court après la mesure de celle-ci.

Dans un mode de réalisation particulier, l'invention a également pour objectif de de fournir un tel dispositif qui permette une prévision de la présence de pollens dans l'air en un point donné.

L'invention a également pour objectif de fournir un procédé de détection de présence de pollen dans l'air, qui permette d'effectuer une telle détection de façon plus rapide et efficace que les procédés connus.

### Exposé de l'invention

Ces objectifs, ainsi que d'autres qui apparaîtront plus clairement par la suite, sont atteints à l'aide d'un dispositif de détection de présence de pollens dans l'air, comprenant une chambre de mesure isolée de la lumière extérieure, des moyens d'entraînement d'un flux d'air à travers cette chambre de mesure, et une source lumineuse émettant un faisceau lumineux dans une direction de propagation passant par le flux d'air, dans la chambre de mesure, ce dispositif comprenant, selon l'invention, au moins quatre capteurs photosensibles aptes à mesurer le flux lumineux diffusé par le flux d'air éclairé, parmi lesquels :
- un premier capteur mesurant le flux lumineux diffusé dans une direction formant un angle compris entre 5° et 25° avec la direction de propagation ;
- un dernier capteur mesurant le flux lumineux diffusé dans une direction formant un angle compris entre 150° et 175° avec la direction de propagation ;
- au moins deux capteurs mesurant chacun le flux lumineux diffusé dans une direction formant un angle compris entre 25° et 150° avec la direction de propagation ;
le dispositif comprenant également une horloge, au moins deux capteurs météorologiques, et au moins un calculateur apte à déterminer la nature d'une particule de pollen présente dans l'air à partir des données mesurées par les capteurs photosensibles, l'horloge et les capteurs météorologiques.

Un tel dispositif permet d'effectuer une détection fiable, efficace et presque instantanée des particules de pollens dans l'air. Il est par ailleurs relativement facile à fabriquer et peu onéreux, ce qui permet d'en équiper un grand nombre de sites.

Avantageusement, le dispositif comprend seulement quatre capteurs photosensibles aptes à mesurer le flux lumineux diffusé par le flux d'air éclairé.

Ces quatre capteurs sont suffisants pour obtenir les mesures permettant de reconnaître de façon satisfaisante la majorité des pollens qui sont connus comme susceptibles de déclencher des allergies. Un dispositif comprenant ces quatre capteurs peut donc, pour un coût modéré, détecter en temps réel la présence dans l'air de grains de pollens, et identifier le type de chaque grain de pollen.

Avantageusement, le premier capteur mesure le flux lumineux diffusé dans une direction formant un angle compris entre 5° et 20° avec la direction de propagation.

Avantageusement, le dernier capteur mesure ledit flux lumineux diffusé dans une direction formant un angle compris entre 150° et 160° avec la direction de propagation.

Ces choix d'angles sont en effet ceux qui ont été identifiés comme permettant les mesures les plus utiles à la détection des pollens.

Selon un mode de réalisation préférentiel, le dispositif comprend une base de données comprenant des données de flux lumineux diffusés par des pollens de différents types, le calculateur étant apte à comparer ces données avec les flux lumineux mesurés par les capteurs photosensibles.

De façon préférentielle, les capteurs météorologiques comprennent au moins deux capteurs choisis parmi :
- un thermomètre,
- un baromètre,
- un hygromètre,
- un anémomètre,
- un pluviomètre et
- un capteur de luminosité.

Avantageusement, les capteurs météorologiques comprennent au moins :
- un thermomètre,
- un baromètre,
- un hygromètre.

Le choix de ces trois capteurs météorologiques permet en effet une mesure efficace des paramètres météorologiques, tout en limitant la complexité et le coût du dispositif.

De façon avantageuse, le dispositif comprend une base de données comprenant des données concernant la probabilité d'apparition de pollens en fonction de la date et de paramètres météorologiques, et le calculateur est apte à comparer ces données avec les données mesurées par l'horloge et les capteurs météorologiques.

Avantageusement, le dispositif comprend au moins un équipement de géolocalisation et une base de données comprenant des données concernant la probabilité d'apparition de pollens en fonction de la géolocalisation, le calculateur étant apte à prendre en compte la géolocalisation pour déterminer la nature d'une particule de pollen.

L'invention concerne également un procédé de détection de présence de pollens dans l'air, comprenant :
- une étape d'illumination d'un flux d'air circulant dans une chambre de mesure, par un flux lumineux orienté dans une direction de propagation ;
- une étape de mesure du flux lumineux diffusé par le flux d'air illuminé, dans au moins quatre directions parmi lesquelles une direction formant un angle compris entre 5° et 25° avec la direction de propagation, une direction formant un angle compris entre 150° et 175° avec la direction de propagation, et au moins deux directions formant chacune un angle compris entre 25° et 150° avec la direction de propagation ;
- une étape de détermination, par un calculateur, de la nature d'une particule de pollen présente dans le flux d'air, à partir des données de flux lumineux diffusé mesurées à l'étape de mesure, de données de datation et de données météorologiques.

### Liste des figures

L'invention sera mieux comprise à la lecture de la description suivante de modes de réalisation préférentiels, donnée à titre de simple exemple figuratif et non limitatif, et accompagnée des figures parmi lesquelles :
[Fig. 1] la figure 1 est une représentation schématique d'un dispositif de détection de pollens selon un mode de réalisation de l'invention ;
[Fig. 2] la figure 2 est une vue en perspective du détecteur de pollens du dispositif représenté par la figure 1 ;
[Fig. 3] la figure 3 est une autre vue en perspective du détecteur de pollens représenté par la figure 2 ;
[Fig. 4] la figure 4 est une vue de dessus du détecteur de pollens représenté par la figure 2 ;
[Fig. 5] la figure 5 est une coupe longitudinale du détecteur de pollens représenté par la figure 2 ;
[Fig. 6] la figure 6 est une coupe transversale du détecteur de pollens représenté par la figure 2 ;
[Fig. 7] la figure 7 est une courbe représentative de la lumière diffusée par deux particules de pollen illuminées, en fonction de la direction de diffusion.

### Description détaillée de modes de réalisation de l'invention

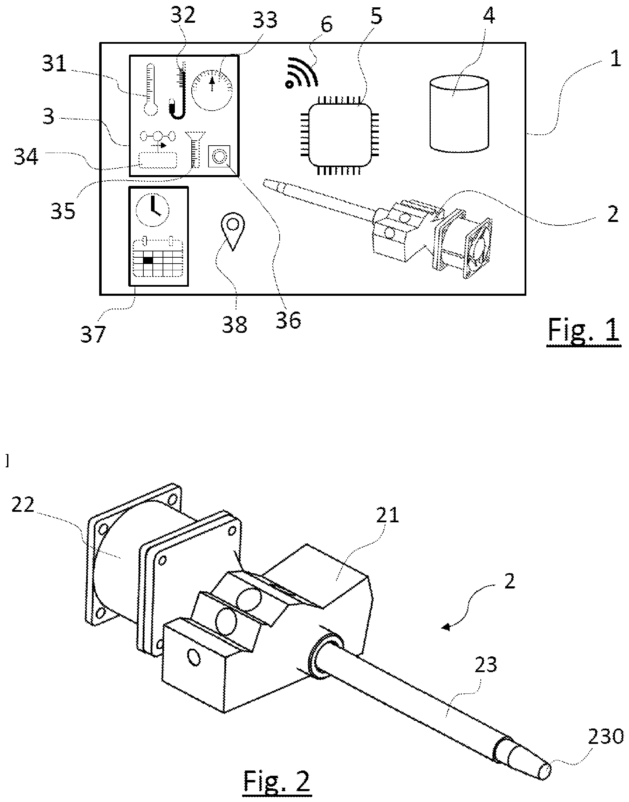

La figure 1 est une représentation schématique d'un dispositif de détection de présence de pollens dans l'air selon un mode de réalisation de l'invention. Ce dispositif 1 comprend plusieurs composants, et notamment un détecteur de pollens 2, capable de détecter et de mesurer la présence de grains de pollen dans l'air. Il comprend également un ensemble 3 de capteurs météorologiques, une horloge 37, un équipement de géolocalisation 38, une base de données 4, un calculateur 5 et un module de transmission 6.

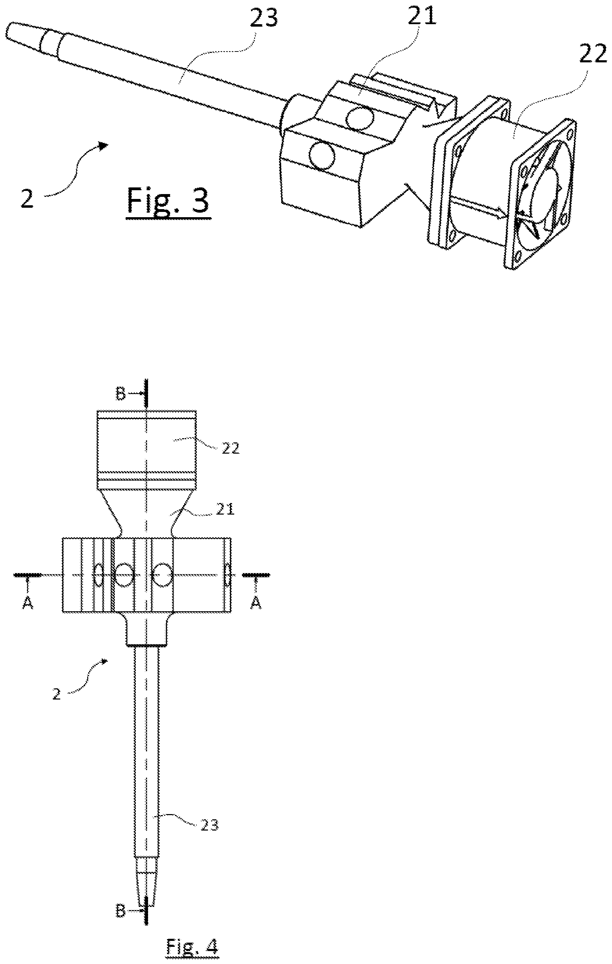

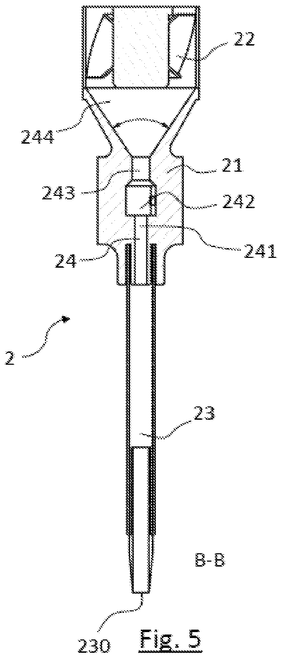

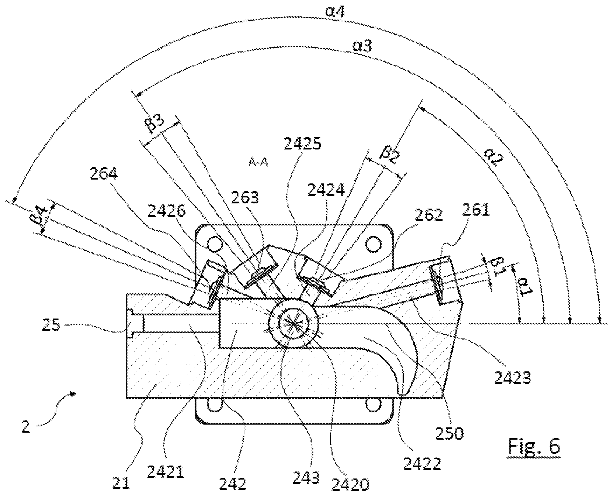

Le détecteur de pollens 2 est représenté, sous des orientations différentes, par les figures 2 à 4, et en vue de coupe par les figures 5 et 6.

Ce détecteur 2 comprend un boîtier de mesure 21 qui est équipé, à l'une de ses extrémités, d'une pompe d'aspiration 22 et, à l'autre de ses extrémités, d'une pipe d'aspiration 23. La pompe d'aspiration 22 et la pipe d'aspiration 23 sont montées aux deux extrémités d'une conduite interne 24 du boîtier de mesure 21. Ainsi, la pompe d'aspiration 22 peut créer une dépression dans cette conduite interne 24 et dans la pipe d'aspiration 23 afin que l'air ambiant soit aspiré par l'extrémité ouverte 230 de la pipe d'aspiration 23, et traverse la pipe d'aspiration 23 et la conduite interne 24 du boîtier de mesure 21 avant d'être refoulé hors du détecteur de pollens 2 par la pompe 22.

De façon avantageuse, dans le mode de réalisation représenté, le boîtier de mesure 21 est monobloc. Il est pour cela réalisé par un procédé de fabrication additive. Cette configuration permet d'éviter que ce boîtier de mesure 21 soit traversé par un plan de coupe, qui pourrait générer un risque d'introduction dans la conduite interne 24 de lumière pouvant parasiter les mesures.

La figure 5 est une vue en coupe longitudinale du détecteur 2, qui montre la conduite interne 24 du boîtier de mesure 21. Cette conduite interne comprend une portion d'entrée 241 sensiblement cylindrique, qui débouche sur la pipe d'aspiration 23 à une première de ses extrémités. À la seconde extrémité de cette portion d'entrée 241, la conduite interne 24 comprend une chambre de mesure 242. Une portion de sortie 243 sensiblement cylindrique débouche sur la chambre de mesure 242, en face de la portion d'entrée 241.

Entre la portion de sortie 243 et la pompe d'aspiration 22, la conduite interne prend la forme d'une portion conique 244, dont une extrémité présente le diamètre de la pompe d'aspiration 22 et l'autre extrémité présente le diamètre de la conduite de sortie 243. De préférence, la pipe d'aspiration 23 et toutes les portions de la conduite interne 24, à l'exception de la chambre de mesure 242, sont centrées sur un même axe longitudinal, appelé par la suite axe longitudinal de la conduite interne 24.

De préférence, la pompe d'aspiration 22 aspire l'air de façon à lui donner une vitesse rapide dans la portion d'entrée 241 et dans la chambre de mesure 242. Ainsi, dans le mode de réalisation représenté, la pompe d'aspiration 22 est configurée pour pomper entre 0,5 m³ et 1 m³ d'air par heure.

La portion d'entrée 241 présente un diamètre relativement faible, de 4 mm dans le mode de réalisation représenté, afin de communiquer une vitesse relativement importante à l'air circulant dans cette portion d'entrée 241 et dans la chambre de mesure 242.

La portion de sortie 243 présente un diamètre légèrement supérieur à celui de la portion d'entrée 241, de 6 mm dans le mode de réalisation représenté, afin de faciliter le passage dans cette portion de sortie 243 des particules présentes dans l'air circulant dans la conduite interne 24, et d'éviter que ces particules ne restent piégées dans la chambre de mesure 242.

La disposition des équipements d'aspiration et de la conduite interne 24 du boîtier de mesure 21 permet donc que la chambre de mesure 242 soit traversée par un flux d'air circulant rapidement, ce flux d'air s'inscrivant sensiblement dans un cylindre d'un diamètre inférieur à 6 mm.

Le boîtier de mesure 21 est avantageusement équipé pour analyser, de façon optique, les particules contenues dans ce flux d'air traversant la chambre de mesure 242.

La figure 6 est une vue de coupe du détecteur 2, suivant un plan transversal à l'axe longitudinal de la conduite interne 24 et passant par la chambre de mesure 242. Cette chambre de mesure 242 est organisée autour d'un point de mesure 2420, qui correspond à l'intersection entre l'axe longitudinal de la conduite interne 24 et le plan de mesure, correspondant au plan de coupe de la figure 6, sur lequel sont alignés les instruments de mesure.

Plusieurs orifices sont prévus dans la chambre de mesure 242 pour recevoir les équipements de mesure. Ainsi, un premier orifice cylindrique 2421, dont l'axe est aligné avec le plan de mesure et avec le point de mesure 2420, est destiné à recevoir un émetteur laser 25, constituant une source lumineuse émettant un faisceau lumineux dans une direction de propagation 250 centrée sur le point de mesure 2420, pour illuminer le flux d'air traversant la chambre de mesure 242. De façon préférentielle, l'ouverture de ce faisceau lumineux est choisie de façon à ce qu'il illumine la totalité de la largeur du flux d'air traversant la chambre 242.

À son extrémité opposée à la conduite 2421, la chambre de mesure 242 présente un orifice fermé 2422, conformé pour constituer un piège à lumière. Ce piège à lumière permet que le faisceau lumineux émis par l'émetteur laser 25 n'interfère pas avec les autres composants du boîtier de mesure 24, après qu'il ait illuminé le flux d'air traversant la chambre 242.

La chambre de mesure 242 présente également quatre orifices cylindriques de mesure, dont les axes sont inclus dans le plan de mesure et se croisent au niveau du point de mesure 2420. Chacun de ces orifices de mesure présente une orientation précise, mesurée sur la figure 6 par rapport à la direction de propagation 250 du faisceau laser, qui correspond à l'axe de l'orifice 2421. Ainsi,
- l'axe d'un premier orifice de mesure 2423, à l'extrémité duquel est placé un capteur photosensible 261, forme un angle α1 = 15° avec la direction de propagation 250 ;
- l'axe d'un deuxième orifice de mesure 2424, à l'extrémité duquel est placé un capteur photosensible 262, forme un angle α2 = 60° avec la direction de propagation 250 ;
- l'axe d'un troisième orifice de mesure 2425, à l'extrémité duquel est placé un capteur photosensible 263, forme un angle α3 = 125° avec la direction de propagation 250 ;
- l'axe d'un quatrième orifice de mesure 2426, à l'extrémité duquel est placé un capteur photosensible 264, forme un angle α4 = 157° avec la direction de propagation 250.

Par ailleurs, chacun de ces orifices de mesure présente des dimensions choisies de façon à offrir une ouverture angulaire déterminée, partant du point de mesure 2420 jusqu'à l'extrémité de l'orifice de mesure, qui est couverte par le capteur photosensible. Cette ouverture angulaire, autour de l'axe de l'orifice de mesure, est choisie pour que le capteur photosensible capte simultanément la lumière émise dans une plage angulaire déterminée. Ainsi,
- l'orifice de mesure 2423 est dimensionné de telle sorte que le capteur photosensible 261 capte la lumière diffusée sur une plage angulaire d'ouverture β1=4,4° et centrée sur l'angle α1 ;
- l'orifice de mesure 2424 est dimensionné de telle sorte que le capteur photosensible 262 capte la lumière diffusée sur une plage angulaire d'ouverture β2=13,5° et centrée sur l'angle α2 ;
- l'orifice de mesure 2425 est dimensionné de telle sorte que le capteur photosensible 263 capte la lumière diffusée sur une plage angulaire d'ouverture β3=10,6° et centrée sur l'angle α3 ;
- l'orifice de mesure 2426 est dimensionné de telle sorte que le capteur photosensible 264 capte la lumière diffusée sur une plage angulaire d'ouverture β4=7,5° et centrée sur l'angle α4.

Il est à noter que, dans d'autres modes de réalisation possibles de l'invention, la position angulaire des différents orifices de mesure peut varier. Il est cependant important qu'un premier orifice de mesure soit centré sur une direction formant un angle faible avec la direction de propagation, de préférence compris entre 5° et 25°, et plus avantageusement compris entre 5° et 20°, et qu'un dernier orifice de mesure soit centré sur une direction formant un angle fort avec la direction de propagation, de préférence compris entre 150° et 175° et plus avantageusement compris entre 150° et 160°.

Entre ces deux orifices de mesure, au moins deux autres orifices de mesure doivent être positionnés à des positions angulaires différentes, par rapport à la direction de propagation. Les positions angulaires de ces orifices de mesure, et donc des capteurs photosensibles, peuvent cependant être différentes de celles qui sont représentées sur la figure 6.

Dans le mode de réalisation représenté, la chambre de mesure 242 présente quatre capteurs photosensibles. Il est également possible de mettre en oeuvre un détecteur dans lequel la chambre de mesure comprenne plus de quatre capteurs photosensibles, par exemple cinq capteurs photosensibles. Dans ce cas, trois orifices comprenant des capteurs photosensibles sont placés à des positions angulaires variées entre le premier orifice centré sur une direction formant un angle compris entre 5° et 25° avec la direction de propagation, et le dernier orifice centré sur une direction formant un angle compris entre 150° et 175° avec la direction de propagation.

Les inventeurs ont cependant déterminé que le choix d'une chambre de mesure comprenant quatre capteurs photosensibles, mesurant chacun la lumière émise dans une direction formant un angle différent avec la direction de propagation, constituait le compromis le plus avantageux. Ces capteurs photosensibles sont en effet suffisants, dans un grand nombre de situations, pour que leurs mesures puissent permettre d'identifier les particules de pollens. Un plus grand nombre de capteurs photosensibles peut permettre d'obtenir des mesures plus précises, mais qui ne permettent d'obtenir que des améliorations relativement faibles pour l'identification des pollens. En revanche, la mise en place d'un plus grand nombre de capteurs photosensibles augmente de façon importante la complexité, et donc le coût, du détecteur de pollen.

Quand le détecteur 2 est en fonctionnement, l'émetteur laser 25 émet un faisceau lumineux, dans la direction de propagation 250, qui illumine le flux d'air traversant la chambre de mesure 242. Quand ce flux d'air ne contient aucune particule, il est transparent et l'intégralité du faisceau laser termine sa trajectoire dans le piège de lumière constitué par l'orifice 2422.

Quand le flux d'air contient une particule, celle-ci est brièvement éclairée par le faisceau lumineux, lors de son passage à proximité du point de mesure 2420. Au moment où elle est éclairée, cette particule va diffuser dans différentes directions la lumière qu'elle reçoit. Le flux de lumière émis lors de cette diffusion varie en fonction de l'angle d'émission, ou angle de diffusion, qui est mesuré par rapport à la direction de propagation du faisceau laser d'éclairage.

Ce flux de lumière est variable en fonction de différents critères, parmi lesquels la puissance de l'éclairage par le faisceau laser, la longueur d'onde de cet éclairage, la forme de la particule éclairée, la couleur de la particule éclairée, la taille de la particule éclairée, l'état de surface de la particule éclairée, l'opacité de la particule éclairée, le caractère réfléchissant de la particule éclairée, etc.

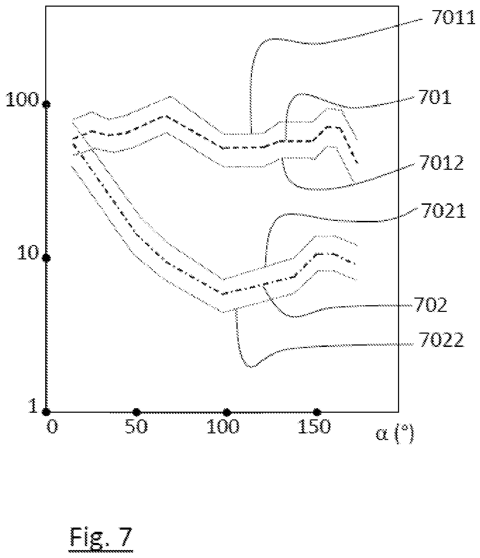

Les inventeurs ont observé que, pour une même illumination, les différents types de particules diffusent un flux lumineux distinct en fonction de l'angle de diffusion. Ainsi, à titre d'exemple, la figure 7 est une courbe représentant le flux lumineux diffusé par deux particules différentes, soumises à la même illumination, en fonction de l'angle de diffusion. La courbe 701 représente ainsi le flux lumineux diffusé par une première particule, qui est une particule de pollen de sapin, et la courbe 702 représente le flux diffusé par une seconde particule, qui est une particule de pollen de maïs.

Sur cette figure, l'axe des abscisses représente les angles α de diffusion, dans lesquels chaque particule émet de la lumière diffusée quand elle est illuminée. L'axe des ordonnées représente le flux lumineux émis par chaque particule dans chacune de ces directions. Cet axe est un axe logarithmique, gradué dans une unité arbitraire. Il est à noter que, pour chaque particule, la valeur du flux est normalisée de façon à présenter une valeur identique à 15°. Les courbes 7011 et 7012 représentent les incertitudes de mesure correspondant à la courbe 701, et les courbes 7021 et 7022 représentent les incertitudes de mesure correspondant à la courbe 702.

Les inventeurs ont ainsi analysé la diffusion lumineuse d'un grand nombre de particules susceptibles d'être présentes dans l'air, et notamment des particules de pollens observées le plus couramment. Ils ont observé que les courbes de diffusion lumineuse, réalisées avec des équipements configurés pour donner des résultats comparables, étaient généralement distinctes selon le type de particules illuminées, et semblables pour des particules illuminées du même type. Ainsi, la courbe de diffusion 701 du pollen de sapin est très différente de la courbe 702 du pollen de maïs.

Par exemple, une particule de pollen d'un premier type de végétal présente systématiquement une courbe de diffusion semblable à celle des autres particules de pollen du même végétal. Bien entendu, les courbes de diffusion de deux particules ne sont pas pour autant exactement identiques. De légères variations peuvent apparaître entre deux particules, ou en fonction de l'orientation de la particule illuminée. Les inventeurs ont cependant obtenu, en analysant un grand nombre de particules de pollens de chaque type, une courbe représentative de la diffusion moyenne des particules de chaque type, associée à des courbes d'incertitudes, qui peut être variable en fonction de l'angle de diffusion.

Quand des particules présentant des courbes de diffusion différentes sont mesurées, il est possible de les discriminer en effectuant une mesure de la lumière diffusée, à des positions angulaires choisies pour que la valeur de diffusion des deux types de particules représente une différence supérieure aux écarts-types. Cependant, les particules pouvant être présentes dans l'air étant très diverses, il est impossible de toutes les discriminer avec une mesure unique.

À partir de l'analyse des courbes de diffusion d'un grand nombre de pollens et d'autres particules, les inventeurs ont déterminé que les mesures de la lumière diffusée effectuées dans quatre plages angulaires différentes suffisaient à identifier les particules de pollens, dans une grande majorité de cas.

Ainsi, un premier capteur photosensible mesurant la lumière diffusée dans une plage angulaire comprise entre 5° et 25° permet de mesurer une valeur du flux lumineux diffusé qui dépend de la taille de la particule, et permet donc de déterminer cette taille. Ce premier capteur permet donc de détecter le passage d'une particule, et ainsi d'incrémenter un compteur assurant le comptage des particules, de déterminer sa taille et de donner la valeur de référence du flux lumineux diffusé, qui servira de base de comparaison pour les mesures du flux lumineux diffusé aux autres angles. Il est à noter que cet angle de mesure pour le premier capteur photosensible est choisi de façon à être le plus faible possible, tout en évitant de recevoir le flux lumineux directement émis par le faisceau lumineux.

La détermination de la taille des particules permet notamment de distinguer les grains de pollens, de taille couramment comprise entre 10 et 100 µm, des particules plus fines telles que les suies ou les poussières. Il a notamment été observé que les particules minérales présentent rarement des tailles supérieures à 10 µm. Elle permet également de distinguer les grains de pollens de taille relativement faible, par exemple les grains de pollen de pariétaire dont la taille est d'environ 15 µm, des grains de pollens de taille plus importante, comme par exemple les grains de pollen de maïs dont la taille est d'environ 100 µm.

Les autres capteurs photosensibles, positionnés pour mesurer la lumière diffusée selon d'autres angles, permettent de mesurer le flux lumineux diffusé par la particule quand elle est illuminée, qui peut être comparé avec le flux lumineux mesuré par le premier capteur, placé à l'angle le plus faible.

Ainsi, dans le mode de réalisation représenté, le second capteur, positionné pour mesurer la lumière diffusée dans une zone angulaire située autour de 60°, le troisième capteur, positionné pour mesurer la lumière diffusée dans une zone angulaire située autour de 125°, et le quatrième capteur, positionné pour mesurer la lumière diffusée dans une zone angulaire située autour de 157°, permettent de déterminer le flux lumineux diffusé par une particule, dans la direction où ils sont positionnés. Il est alors possible de déterminer le rapport entre le flux lumineux diffusé dans chacune de ces directions et le flux lumineux diffusé à 15°, et de comparer ces rapports avec les courbes de diffusion des différents types de particules de taille comparable, pour déterminer de quelles courbes ces rapports se rapprochent le plus.

Ainsi, la combinaison des mesures dans ces quatre zones angulaires permet, dans un grand nombre de cas, d'identifier la nature d'une particule de pollen. Il est à noter qu'il est particulièrement important que l'un de ces capteurs soit situé dans une zone angulaire comprise entre 150° et 175°, et plus avantageusement entre 150° et 160°. En effet, les inventeurs ont déterminé que plusieurs particules, notamment de pollens, présentaient des courbes de diffusion très semblables pour des angles de diffusion inférieurs à 150°, mais qui divergeaient clairement pour des angles de diffusion compris entre 150° et 160°. Un capteur photosensible situé dans cette zone angulaire permet donc de déterminer le rapport entre le flux lumineux diffusé dans cette zone angulaire et le flux lumineux diffusé à 15°, pour distinguer les particules dont la courbe de diffusion est proche en deçà de 150°.

Ces données mesurées par le détecteur 2 sont transmises au calculateur 5 du dispositif de détection 1, qui compare, lors de l'illumination de chaque particule, les valeurs du flux lumineux diffusé mesurées par chacun des quatre capteurs photosensibles avec des données de flux lumineux diffusés typiques pour chaque type de pollen, qui sont stockées dans la base de données 4. Dans un grand nombre de cas, cette comparaison permet au calculateur 5 de déterminer la nature du grain de pollen qui a été illuminé.

Dans certains cas, cependant, cette comparaison est insuffisante pour déterminer la nature du grain de pollen avec un niveau de fiabilité suffisant. Cela peut arriver, par exemple, quand deux types de pollens différents présentent des courbes très proches dans les directions de diffusion dans lesquelles le flux lumineux est mesuré, et que les valeurs mesurées pour une particule sont susceptibles de correspondre à plusieurs types de pollens.

Pour permettre l'identification de la particule illuminée, le dispositif de détection 1 comprend un jeu de capteurs météorologiques 3. Dans le mode de réalisation représenté, ce jeu de capteurs météorologiques 3 comprend un thermomètre 31, une un baromètre 32, un hygromètre 33, un anémomètre 34, un pluviomètre 35 et un capteur de luminosité 36. Dans d'autres modes de réalisation, le jeu de capteurs météorologiques 3 peut comprendre moins de capteurs, ou d'autres types de capteurs. Un jeu de capteurs météorologiques à la fois efficace, compact et peu coûteux peut ainsi comprendre uniquement un thermomètre, un baromètre et un hygromètre.

Le dispositif de détection 1 comprend également, de façon avantageuse, une horloge 37, permettant de connaître l'heure et la date, appelées par la suite données de datation, et un équipement de géolocalisation 38.

Les capteurs météorologiques, l'horloge et l'équipement de géolocalisation 38 peuvent transmettre les données qu'ils mesurent au calculateur 5. Ce calculateur 5 peut comparer ces données à des données de référence stockées dans la base de données 4, telles que des données de référence concernant les conditions d'apparition des différents types de pollens. Il peut par exemple comparer les données de datation fournie par l'horloge 37 avec un calendrier de l'apparition des différents types de pollens. Ce calendrier peut lui-même être choisi par le calculateur 5, parmi plusieurs calendriers disponibles, en fonction des données de géolocalisation mesurées par l'équipement de géolocalisation 38, pour que ce calendrier corresponde au mieux aux conditions climatiques, et aux types de végétaux connus dans la zone dans laquelle se trouve le dispositif de détection 1.

Le calculateur 5 peut également comparer les données météorologiques mesurées par les capteurs du jeu de capteurs météorologiques 3 avec des données connues concernant l'apparition de chaque type de pollen. Il est par exemple connu, notamment par l'étude de K. Laaidi, M. Laaidi et J.-P. Besancenot, 1997 : POLLENS, POLLINOSES ET MÉTÉOROLOGIE, La Météorologie 8e série - n° 20, 41-56, que les pollens de bouleau apparaissent à Paris quand la somme de 260°C.jour au-dessus de 3°C est cumulée à partir du 11 février. La base de données 4 contient avantageusement de telles informations issues notamment des publications scientifiques.

La comparaison, par le calculateur 5, de ces informations et des mesures obtenues du jeu de capteurs environnementaux 3 permet avantageusement de déterminer une probabilité d'apparition de chaque type de pollen. Ainsi, dans le cas où l'illumination d'une particule entraîne la mesure, par le détecteur de pollen 2, de valeurs de flux lumineux diffusés pouvant correspondre à plusieurs types de pollens, la probabilité d'apparition associée à chacun de ces types de pollens permet, dans un grand nombre de cas, de déterminer avec un niveau de fiabilité suffisant le type de cette particule.

Avantageusement, le calculateur 5 peut ainsi comprendre un programme permettant de prendre en compte à la fois les informations des capteurs photosensibles, des capteurs météorologiques, de l'horloge et de l'équipement de géolocalisation, pour déterminer la nature de la particule de pollen détectée.

Selon un mode de réalisation avantageux, le calculateur 5 peut être équipé d'un programme d'apprentissage automatique, usuellement désigné sous le nom de programme de « Machine Learning », apte à identifier des corrélations entre les données mesurées par différents capteurs du dispositif de détection 1 et le type de particules de pollens détectées par le détecteur 2. Un tel programme d'apprentissage automatique peut définir des règles d'identification permettant d'accélérer, et de rendre plus fiable, l'identification des particules détectées par le détecteur 2. Il peut également permettre, dans certains cas, de prédire l'apparition de certains types de pollens, avant même leur détection par le détecteur 2, à partir de données mesurées par les capteurs météorologiques, l'horloge 37 et l'équipement de géolocalisation 38.

Le dispositif de détection 1 est avantageusement équipé d'un module de communication 6. Ce module permet l'envoi à un serveur distant d'informations sur le type et la quantité des grains de pollens détectés. Le serveur distant peut ainsi collecter, en temps réel, des données de mesure de pollens à l'endroit où est placé le dispositif de détection 1.

Dans un mode de réalisation avantageux, le serveur distant peut également communiquer au dispositif de détection 1 des données météorologiques, par exemple des données de prévisions météorologiques, et des données de mesure ou de présence de pollens mesurées par d'autres dispositifs de détection de pollens, du même type ou de type différent. De telles données peuvent avantageusement être utilisées par le calculateur 5, au même titre que les données provenant des capteurs du dispositif de détection 1, pour déterminer la probabilité d'apparition de chaque type de pollen, afin de contribuer à l'identification d'une particule illuminée dans le capteur 2 ou de prédire l'apparition de pollens.

## Revendications

1. Dispositif de détection de présence de pollens dans l'air, comprenant une chambre de mesure (242) isolée de la lumière extérieure, des moyens d'entraînement d'un flux d'air à travers ladite chambre de mesure (242), et une source lumineuse (25) émettant un faisceau lumineux dans une direction de propagation (250) passant par ledit flux d'air, dans ladite chambre de mesure (242),
**caractérisé en ce qu'**il comprend au moins quatre capteurs photosensibles (261, 262, 263, 264) aptes à mesurer le flux lumineux diffusé par ledit flux d'air éclairé, parmi lesquels :
- un premier capteur (261) mesurant ledit flux lumineux diffusé dans une direction formant un angle compris entre 5° et 25° avec ladite direction de propagation (250) ;
- un dernier capteur (264) mesurant ledit flux lumineux diffusé dans une direction formant un angle compris entre 150° et 175° avec ladite direction de propagation (250) ;
- au moins deux capteurs (262, 263) mesurant chacun ledit flux lumineux diffusé dans une direction formant un angle compris entre 25° et 150° avec ladite direction de propagation (250) ;
**et en ce qu'il comprend** une horloge (37), au moins deux capteurs météorologiques, et au moins un calculateur (5) apte à déterminer la nature d'une particule de pollen présente dans l'air à partir des données mesurées par lesdits capteurs photosensibles (261, 262, 263, 264), ladite horloge (37) et lesdits capteurs météorologiques.

2. Dispositif de détection selon la revendication précédente, **caractérisé en ce qu'**il comprend seulement quatre capteurs photosensibles (261, 262, 263, 264) aptes à mesurer le flux lumineux diffusé par ledit flux d'air éclairé.

3. Dispositif de détection selon l'une quelconque des revendications précédentes, **caractérisé en ce que** ledit premier capteur (261) mesure ledit flux lumineux diffusé dans une direction formant un angle compris entre 5° et 20° avec ladite direction de propagation (250).

4. Dispositif de détection selon l'une quelconque des revendications précédentes, **caractérisé en ce que** ledit dernier capteur (264) mesure ledit flux lumineux diffusé dans une direction formant un angle compris entre 150° et 160° avec ladite direction de propagation (250).

5. Dispositif de détection selon l'une quelconque des revendications précédentes, **caractérisé en ce qu'**il comprend une base de données (4) comprenant des données de flux lumineux diffusés par des pollens de différents types, ledit calculateur (5) étant apte à comparer lesdites données avec les flux lumineux mesurés par lesdits capteurs photosensibles (261, 262, 263, 264).

6. Dispositif de détection selon l'une quelconque des revendications précédentes, **caractérisé en ce que** lesdits capteurs météorologiques comprennent au moins deux capteurs choisis parmi :
- un thermomètre (31),
- un baromètre (32),
- un hygromètre (33),
- un anémomètre (34),
- un pluviomètre (35) et
- un capteur de luminosité (36).

7. Dispositif de détection selon la revendication précédente, **caractérisé en ce que** lesdits capteurs météorologiques comprennent au moins :
- un thermomètre (31),
- un baromètre (32),
- un hygromètre (33).

8. Dispositif de détection selon l'une quelconque des revendications précédentes, **caractérisé en ce qu'**il comprend une base de données (4) comprenant des données concernant la probabilité d'apparition de pollens en fonction de la date et de paramètres météorologiques, ledit calculateur (5) étant apte à comparer lesdites données avec les données mesurées par ladite horloge (37) et lesdits capteurs météorologiques.

9. Dispositif de détection selon l'une quelconque des revendications précédentes, **caractérisé en ce qu'**il comprend au moins un équipement de géolocalisation (38) et une base de données (4) comprenant des données concernant la probabilité d'apparition de pollens en fonction de la géolocalisation, ledit calculateur (5) étant apte à prendre en compte ladite géolocalisation pour déterminer la nature d'une particule de pollen.

10. Procédé de détection de présence de pollens dans l'air, comprenant :
- une étape d'illumination d'un flux d'air circulant dans une chambre de mesure (242), par un flux lumineux orienté dans une direction de propagation (250) ;
- une étape de mesure du flux lumineux diffusé par ledit flux d'air illuminé, dans au moins quatre directions parmi lesquelles une direction formant un angle compris entre 5° et 25° avec ladite direction de propagation (250), une direction formant un angle compris entre 150° et 175° avec ladite direction de propagation (250), et au moins deux directions formant chacune un angle compris entre 25° et 150° avec ladite direction de propagation (250) ;
- une étape de détermination, par un calculateur (5), de la nature d'une particule de pollen présente dans ledit flux d'air, à partir des données de flux lumineux diffusés mesurées à ladite étape de mesure, de données de datation et de données météorologiques.

## Patentansprüche

1. Vorrichtung zum Erfassen des Vorhandenseins von Pollen in der Luft, umfassend eine vom Außenlicht isolierte Messkammer (242), Mittel zum Antrieb eines Luftstroms durch die Messkammer (242), und eine Lichtquelle (25), die einen Lichtstrahl in einer Ausbreitungsrichtung (250) durch den Luftstrom in die Messkammer (242) emittiert,
**dadurch gekennzeichnet, dass** sie mindestens vier lichtempfindliche Sensoren (261, 262, 263, 264) umfasst, die in der Lage sind, den Lichtstrom zu messen, der durch den beleuchteten Luftstrom gestreut wird, darunter:
- einen ersten Sensor (261), der den gestreuten Lichtstrom in einer Richtung misst, die einen Winkel zwischen 5° und 25° mit der Ausbreitungsrichtung (250) bildet;
- einen letzten Sensor (264), der den gestreuten Lichtstrom in einer Richtung misst, die einen Winkel zwischen 150° und 175° mit der Ausbreitungsrichtung (250) bildet;
- mindestens zwei Sensoren (262, 263), die jeweils den Lichtstrom messen, der in einer Richtung gestreut wird, die einen Winkel zwischen 25° und 150° mit der Ausbreitungsrichtung (250) bildet;
und **dadurch, dass** sie eine Uhr (37), mindestens zwei Wettersensoren und mindestens einen Rechner (5) umfasst, der in der Lage ist, die Art eines in der Luft vorhandenen Pollenpartikels anhand der von den lichtempfindlichen Sensoren (261, 262, 263, 264), der Uhr (37) und den Wettersensoren gemessenen Daten zu bestimmen.

2. Erfassungsvorrichtung nach dem vorstehenden Anspruch,
**dadurch gekennzeichnet, dass** sie nur vier lichtempfindliche Sensoren (261, 262, 263, 264) umfasst, die in der Lage sind, den Lichtstrom zu messen, der durch den beleuchteten Luftstrom gestreut wird.

3. Erfassungsvorrichtung nach einem der vorstehenden Ansprüche,
**dadurch gekennzeichnet, dass** der erste Sensor (261) den gestreuten Lichtstrom in einer Richtung misst, die einen Winkel zwischen 5° und 20° mit der Ausbreitungsrichtung (250) bildet.

4. Erfassungsvorrichtung nach einem der vorstehenden Ansprüche,
**dadurch gekennzeichnet, dass** der letzte Sensor (264) den gestreuten Lichtstrom in einer Richtung misst, die einen Winkel zwischen 150° und 160° mit der Ausbreitungsrichtung (250) bildet.

5. Erfassungsvorrichtung nach einem der vorstehenden Ansprüche,
**dadurch gekennzeichnet, dass** sie eine Datenbank (4) umfasst, die Daten von Lichtströmen umfasst, die von Pollen unterschiedlicher Arten gestreut werden, wobei der Rechner (5) in der Lage ist, diese Daten mit den von den lichtempfindlichen Sensoren (261, 262, 263, 264) gemessenen Lichtströmen zu vergleichen.

6. Erfassungsvorrichtung nach einem der vorstehenden Ansprüche, **dadurch gekennzeichnet, dass** die Wettersensoren mindestens zwei Sensoren umfassen, die ausgewählt sind aus:
- einem Thermometer (31),
- einem Barometer (32),
- einem Hygrometer (33),
- einem Anemometer (34),
- einem Pluviometer (35) und
- einem Helligkeitssensor (36).

7. Erfassungsvorrichtung nach dem vorstehenden Anspruch,
**dadurch gekennzeichnet, dass** die Wettersensoren mindestens Folgendes umfassen:
- ein Thermometer (31),
- ein Barometer (32),
- ein Hygrometer (33).

8. Erfassungsvorrichtung nach einem der vorstehenden Ansprüche, **dadurch gekennzeichnet, dass** sie eine Datenbank (4) umfasst, die Daten bezüglich der Wahrscheinlichkeit des Auftretens von Pollen in Abhängigkeit vom Datum und von meteorologischen Parametern umfasst, wobei der Rechner (5) in der Lage ist, diese Daten mit den von der Uhr (37) und den Wettersensoren gemessenen Daten zu vergleichen.

9. Erfassungsvorrichtung nach einem der vorstehenden Ansprüche,
**dadurch gekennzeichnet, dass** sie mindestens eine Geolokalisierungsausrüstung (38) und eine Datenbank (4) umfasst, die Daten bezüglich der Wahrscheinlichkeit des Auftretens von Pollen in Abhängigkeit von der Geolokalisierung umfasst, wobei der Rechner (5) in der Lage ist, die Geolokalisierung zu berücksichtigen, um die Art eines Pollenpartikels zu bestimmen.

10. Verfahren zum Nachweis des Vorhandenseins von Pollen in der Luft, umfassend:
- einen Schritt der Beleuchtung eines Luftstroms, der in einer Messkammer (242) zirkuliert, durch einen Lichtstrom, der in eine Ausbreitungsrichtung (250) ausgerichtet ist;
- einen Schritt der Messung des Lichtstroms, der durch den beleuchteten Luftstrom gestreut wird, in mindestens vier Richtungen, darunter eine Richtung, die einen Winkel zwischen 5° und 25° mit der Ausbreitungsrichtung (250) bildet, eine Richtung, die einen Winkel zwischen 150° und 175° mit der Ausbreitungsrichtung (250) bildet, und mindestens zwei Richtungen, die jeweils einen Winkel zwischen 25° und 150° mit der Ausbreitungsrichtung (250) bilden;
- einen Schritt der Bestimmung, durch einen Rechner (5), der Art eines Pollenpartikels, der in dem Luftstrom vorhanden ist, anhand der Daten des gestreuten Lichtstroms, die in dem Messschritt gemessen wurden, der Datierungsdaten und der meteorologischen Daten.

## Claims

1. Device for detecting the presence of pollen in the air, comprising a measuring chamber (242) isolated from external light, means for driving an air flow through said measuring chamber (242), and a light source (25) emitting a light beam in a direction of propagation (250) through said air flow, into said measuring chamber (242),
**characterized in that** it comprises at least four photosensitive sensors (261, 262, 263, 264) capable of measuring the luminous flux diffused by said illuminated air flow, including:
- a first sensor (261) measuring said luminous flux diffused in a direction forming an angle of between 5° and 25° with said direction of propagation (250);
- a last sensor (264) measuring said luminous flux diffused in a direction forming an angle of between 150° and 175° with said direction of propagation (250);
- at least two sensors (262, 263) each measuring said luminous flux diffused in a direction forming an angle of between 25° and 150° with said direction of propagation (250);
**and in that it comprises** a clock (37), at least two meteorological sensors, and at least one computer (5) capable of determining the nature of a pollen particle present in the air from the data measured by said photosensitive sensors (261, 262, 263, 264), said clock (37) and said meteorological sensors.

2. Detection device according to the preceding claim, **characterized in that** it comprises four photosensitive sensors (261, 262, 263, 264) capable of measuring the luminous flux diffused by said illuminated air flow.

3. Detection device according to one of the preceding claims, **characterized in that** said first sensor (261) measures said luminous flux diffused in a direction forming an angle comprised between 5° and 20° with said direction of propagation (250).

4. Detection device according to one of the preceding claims, **characterized in that** said last sensor (264) measures said luminous flux diffused in a direction forming an angle comprised between 150° and 160° with said direction of propagation (250).

5. Detection device according to one of the preceding claims, **characterized in that** it comprises a database (4) comprising luminous flux data diffused by pollen of different types, said computer (5) being able to compare said data with the luminous fluxes measured by said photosensitive sensors (261, 262, 263, 264).

6. Detection device according to one of the preceding claims, **characterized in that** said meteorological sensors comprise at least two sensors chosen from:
- a thermometer (31),
- a barometer (32),
- a hygrometer (33),
- an anemometer (34),
- a rain gauge (35), and
- a light sensor (36).

7. Detection device according to the preceding claim, **characterized in that** said meteorological sensors comprise at least:
- a thermometer (31),
- a barometer (32),
- a hygrometer (33).

8. Detection device according to one of the preceding claims, **characterized in that** it comprises a database (4) comprising data relating to the probability of the appearance of pollen as a function of the date and meteorological parameters, said computer (5) being capable of comparing said data with the data measured by said clock (37) and said meteorological sensors.

9. Detection device according to one of the preceding claims, **characterized in that** it comprises at least one geolocation device (38) and a database (4) comprising data concerning the probability of the appearance of pollen as a function of geolocation, said computer (5) being able to take said geolocation into account to determine the nature of a pollen particle.

10. Method for detecting the presence of pollen in the air, comprising:
- a step of illuminating an air flow circulating in a measuring chamber (242), by a luminous flux oriented in a direction of propagation (250);
- a step of measuring the luminous flux diffused by said illuminated air flow, in at least four directions, including a direction forming an angle of between 5° and 25° with said direction of propagation (250), a direction forming an angle of between 150° and 175° with said direction of propagation (250), and at least two directions each forming an angle of between 25° and 150° with said direction of propagation (250);
- a step of determining, by a computer (5), the nature of a pollen particle present in said air flow, from the diffused luminous flux data measured in said measuring step, dating data and meteorological data.
